# EUROPEAN PATENT APPLICATION

(11) **EP 4 321 973 A1**
(43) Date of publication of application: **14.02.2024**
(21) Application number: 22784300.0
(22) Date of filing: 13.01.2022
(51) Int. Cl.: G06F 3/01

(54) **INFORMATION PROCESSING METHOD, INFORMATION PROCESSING DEVICE, AND PROGRAM**

(30) Priority: 08.04.2021 JP 2021065822
(71) Applicant: Sony Group Corporation, Tokyo 108-0075 (JP)
(72) Inventor: ISHII, Tamotsu, Tokyo 108-0075 (JP); MOCHIZUKI, Keita, Tokyo 108-0075 (JP); SATO, Tetsuro, Tokyo 108-0075 (JP)
(74) Representative: MFG Patentanwälte Meyer-Wildhagen Meggle-Freund Gerhard PartG mbB
(86) International application number: PCT/JP2022/000903
(87) International publication number: WO 2022/215313

(57) **Abstract**

To improve convenience for a user regarding correction of a drift error.

An information processing method includes performing an estimation of posture information of a moving body including a first rotation angle around an axis in a gravity direction and a second rotation angle around an axis orthogonal to the gravity direction on the basis of an output from a gyro sensor attached to the moving body; and performing a correction of the first rotation angle in a case where a condition regarding the second rotation angle is satisfied.

## Description

### TECHNICAL FIELD

The present disclosure relates to an information processing method, an information processing apparatus, and a program.

### BACKGROUND ART

In recent years, a motion capture technology for acquiring motion information indicating a motion of a user has been actively developed. The acquired motion information is, for example, used for form improvement in sports, or used for applications such as virtual reality (VR) or augmented reality (AR). Furthermore, an avatar video imitating the motion of the user is generated using the acquired motion information, and also the distribution of the avatar video is conducted.

Note that as a method for realizing the motion capture technology, an optical method using a marker, a sensor method using a gyro sensor, an acceleration sensor, and the like, a camera method for analyzing a video, and the like are known. For example, Patent Document 1 discloses a motion capture technology realized by a sensor method.

### CITATION LIST

### PATENT DOCUMENT

Patent Document 1: WO 2019/203188 A

### SUMMARY OF THE INVENTION

### PROBLEMS TO BE SOLVED BY THE INVENTION

In the above-described sensor method, drift errors are accumulated due to use for a long time or motion of the user, and the avatar video may look unnatural. In a case where the drift errors are accumulated in this manner, a design of performing a process for reducing the influence of the drift errors on the basis of determinations and operations by the user can be considered, but the design has room for improvement from the viewpoint of user convenience.

Therefore, the present disclosure proposes a new and improved information processing method, an information processing apparatus, and a program capable of improving user convenience regarding correction of the drift error.

### SOLUTIONS TO PROBLEMS

According to the present disclosure, provided is an information processing method including: performing an estimation of posture information of a moving body including a first rotation angle around an axis in a gravity direction and a second rotation angle around an axis orthogonal to the gravity direction on the basis of an output from a gyro sensor attached to the moving body; and performing a correction of the first rotation angle in a case where a condition regarding the second rotation angle is satisfied.

Furthermore, according to the present disclosure, provided is an information processing apparatus including: an estimation unit configured to estimate posture information of a moving body including a first rotation angle around an axis in a gravity direction and a second rotation angle around an axis orthogonal to the gravity direction on the basis of an output from a gyro sensor attached to the moving body; and a rotation angle correction unit configured to correct the first rotation angle in a case where a condition regarding the second rotation angle is satisfied.

Furthermore, according to the present disclosure, provided is a program for causing a computer to function as: an estimation unit configured to estimate posture information of a moving body including a first rotation angle around an axis in a gravity direction and a second rotation angle around an axis orthogonal to the gravity direction on the basis of an output from a gyro sensor attached to the moving body; and a rotation angle correction unit configured to correct the first rotation angle in a case where a condition regarding the second rotation angle is satisfied.

### BRIEF DESCRIPTION OF DRAWINGS

Fig. 1 is an explanatory diagram showing an information processing system according to an embodiment of the present disclosure.
Fig. 2 is an explanatory diagram showing a specific example of an avatar video V displayed on a viewing user terminal 40.
Fig. 3 is an explanatory diagram showing a configuration of a distribution user terminal 20 according to an embodiment of the present disclosure.
Fig. 4 is an explanatory diagram showing functions of a base tool 250.
Fig. 5 is an explanatory diagram showing a yaw angle, a pitch angle, and a roll angle.
Fig. 6 is an explanatory diagram showing a specific example of posture information stored in a first storage unit 253.
Fig. 7 is an explanatory diagram showing a specific example of generation of skeleton data.
Fig. 8 is an explanatory diagram showing functions of an application unit 260.
Fig. 9 is an explanatory diagram showing a specific example of a guidance screen.
Fig. 10 is an explanatory diagram showing a specific example of an avatar display screen.
Fig. 11 is a flowchart showing a flow up to registration of posture information in the information processing system according to the embodiment of the present disclosure.
Fig. 12 is a flowchart showing correction processing A performed in a case where automatic correction is enabled.
Fig. 13 is a flowchart showing correction processing B performed in a case where automatic correction is not enabled.
Fig. 14 is a flowchart showing a registration operation according to a first modification.
Fig. 15 is an explanatory diagram showing a second configuration example of the information processing system.
Fig. 16 is an explanatory diagram showing a third configuration example of the information processing system.
Fig. 17 is an explanatory diagram showing a fourth configuration example of the information processing system.
Fig. 18 is an explanatory diagram showing a fifth configuration example of the information processing system.
Fig. 19 is an explanatory diagram showing a sixth configuration example of the information processing system.
Fig. 20 is a block diagram showing a hardware configuration of the distribution user terminal 20.

### MODE FOR CARRYING OUT THE INVENTION

Hereinafter, preferred embodiments of the present disclosure will be described in detail with reference to the accompanying drawings. Note that, in the present specification and the drawings, components having substantially the same functional configuration are denoted by the same reference numerals, and redundant explanations are omitted.

Furthermore, the "mode for carrying out the invention" is described according to the order of items described below.
1. Outline of information processing system
2. Configuration of distribution user terminal
2-1. Overall configuration
2-2. Functions of base tool
2-3. Functions of application unit
3. Operation
4. Summary
5. Modifications
4-1. First modification
4-2. Second modification
6. Other configuration examples of information processing system
5-1. Second configuration example
5-2. Third configuration example
5-3. Fourth configuration example
5-4. Fifth configuration example
5-5. Sixth configuration example
7. Hardware configuration
8. Supplement

### <<1. Outline of information processing system>>

In order to visualize motion information of a moving body such as a human or an animal, for example, skeleton data expressed by a skeleton structure indicating a body structure is used. The skeleton data includes information on parts and bones that are line segments connecting the parts. Note that the parts in the skeleton structure corresponds to, for example, an end part, a joint part, or the like of the body. Furthermore, the bones in the skeleton structure may correspond to, for example, human bones, but the positions and the number of the bones do not necessarily need to be matched with the actual human skeleton.

The positions of the parts in the skeleton data can be acquired by various motion capture technologies. For example, there are a camera-type technology in which a marker is attached to each body part and the position of the marker is acquired using an external camera or the like, and a sensor-type technology in which a motion sensor is attached to a body part and position information of the motion sensor is acquired on the basis of sensor data acquired by the motion sensor.

Furthermore, the skeleton data has various uses. For example, the time-series data of the skeleton data is, for example, used for form improvement in sports, or used for applications such as virtual reality (VR) or augmented reality (AR). Furthermore, an avatar video imitating the motion of the user is generated using the time-series data of the skeleton data, and also the distribution of the avatar video is conducted.

Hereinafter, as an embodiment of the present disclosure, a configuration example of an information processing system that generates skeleton data using a motion sensor such as an acceleration sensor and a gyro sensor and distributes an avatar video on the basis of the skeleton data will be described. Note that, although a human will be mainly described below as an example of a moving body, the embodiment of the present disclosure can be similarly applicable to other moving bodies such as an animal and a robot.

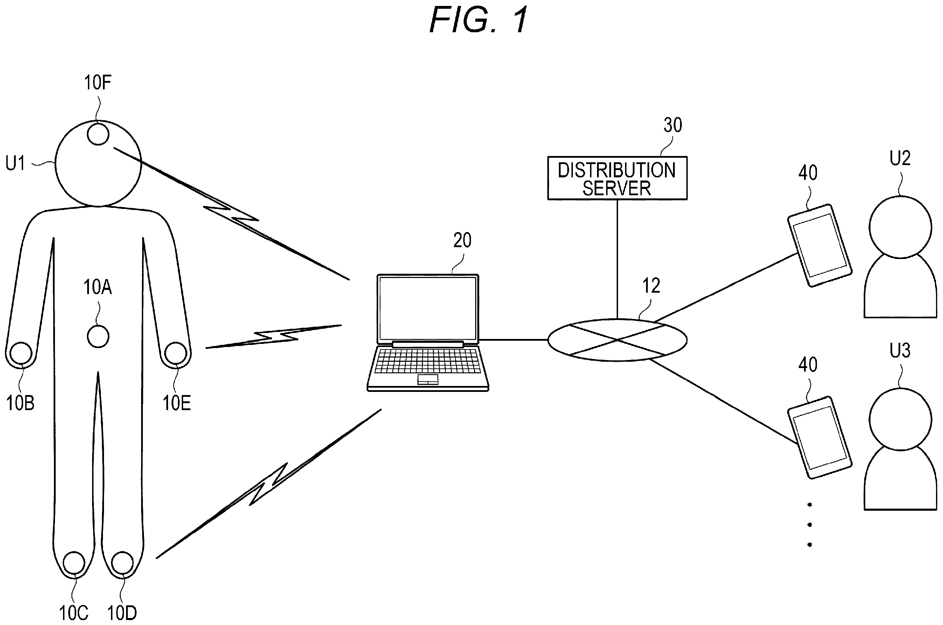

Fig. 1 is an explanatory diagram showing an information processing system according to an embodiment of the present disclosure. As shown in Fig. 1, the information processing system according to the embodiment of the present disclosure includes six sensor devices 10A to 10F, a distribution user terminal 20, a distribution server 30, and a viewing user terminal 40. A user U1 shown in Fig. 1 is a distribution user who distributes an avatar video, and users U2 and U3 are viewing users who view the avatar video.

The distribution user terminal 20, the distribution server 30, and the viewing user terminal 40 are connected via a network 12. The network 12 is a wired or wireless transmission path of information transmitted from a device connected to the network 12. For example, the network 12 may include a public network such as the Internet, a telephone network, or a satellite communication network, various local area networks (LANs) including Ethernet (registered trademark), a wide area network (WAN), and the like. Furthermore, the network 12 may include a dedicated line network such as Internet Protocol-Virtual Private Network (IP-VPN).

### (Sensor device 10)

The sensor device 10 includes, for example, an inertial measurement unit (IMU) such as an acceleration sensor that acquires acceleration or a gyro sensor (angular velocity sensor) that acquires angular velocity. Furthermore, the sensor device 10 may include a sensor such as a geomagnetism sensor, an ultrasonic sensor, or an atmospheric pressure sensor.

It is desirable that the sensor devices 10A to 10F are attached to a joint part (for example, the waist or the head) that serves as a reference of the body or to the vicinity of an end of the body (the wrists, the ankles, the head, and the like). In the example shown in Fig. 1, the sensor device 10A is worn on the waist of the distribution user U1, the sensor devices 10B and 10E are worn on the wrists, the sensor devices 10C and 10D are worn on the ankles, and the sensor device 10F is worn on the head. Note that, hereinafter, there may be a case where the body part to which the sensor device 10 is attached may also be referred to as an attachment part. Furthermore, the number and attachment positions (positions of attachment parts) of the sensor devices 10 are not limited to the example shown in Fig. 1, and the number of sensor devices 10 attached to the distribution user U1 may be added or reduced.

Such a sensor device 10 acquires acceleration, angular velocity, or the like of the attachment part as sensor data, and transmits the sensor data to the distribution user terminal 20.

### (Distribution user terminal 20)

The distribution user terminal 20 is an example of an information processing apparatus used by the distribution user U1. The distribution user terminal 20 receives the sensor data from the sensor device 10, and generates an avatar video of the distribution user U1 using the received sensor data. Although details will be described later, the distribution user terminal 20 acquires attachment part information indicating the position and the posture of each attachment part on the basis of the sensor data, and generates skeleton data including the position information and the posture information of each part in the skeleton structure on the basis of the attachment part information. Moreover, the distribution user terminal 20 generates the avatar video having the posture indicated by the skeleton data. The distribution user terminal 20 transmits the generated avatar video to the distribution server 30, and requests the distribution server 30 to distribute the avatar video.

Note that, in Fig. 1, a notebook personal computer (PC) is shown as the distribution user terminal 20, but the distribution user terminal 20 may be other information processing apparatus such as a smartphone and a desktop PC.

### (Distribution server 30)

The distribution server 30 distributes the avatar video to the viewing user terminal 40 on the basis of a request from the distribution user terminal 20. Although Fig. 1 shows one distribution server 30 that realizes a distribution service provided by a certain business operator, there may be a plurality of business operators and a plurality of distribution servers 30 that provide distribution services. In this case, the distribution user terminal 20 can request the distribution server 30 that provides the distribution service designated by the distribution user U1 to distribute the avatar video.

### (Viewing user terminal 40)

The viewing user terminal 40 is an information processing apparatus used by the viewing user (for example, the user U2 and the user U3 shown in Fig. 1). The viewing user terminal 40 includes a display unit that displays various screens, an operation unit that detects an operation of the viewing user, and a control unit that controls the overall operation of the viewing user terminal 40. For example, the viewing user terminal 40 requests the distribution server 30 to distribute the avatar video of the distribution user U1 on the basis of the operation of the viewing user, and displays the avatar video distributed from the distribution server 30.

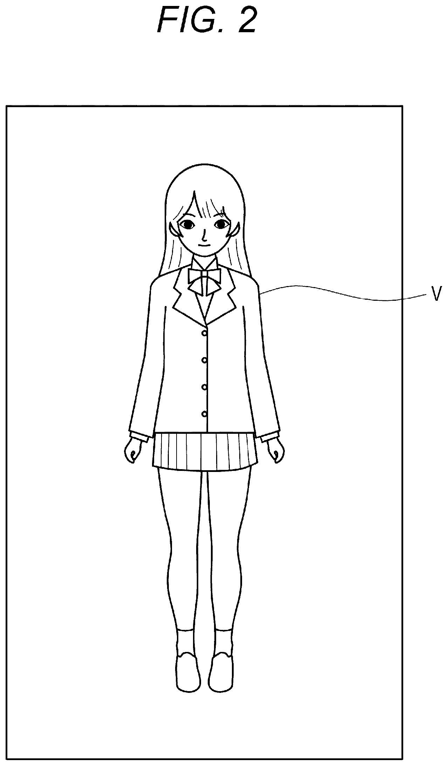

Fig. 2 is an explanatory diagram showing a specific example of an avatar video V displayed on the viewing user terminal 40. As shown in Fig. 2, for example, a video of a two-dimensional character is displayed as the avatar video V on the viewing user terminal 40. The posture of the distribution user U1 is reflected on the posture of the avatar video V. That is, the avatar video V changes in accordance with the motion of the distribution user U1.

### (Background)

In the above-described motion capture technology realized using a motion sensor, there may be a case where drift errors accumulate due to use for a long time or motion of the user, and the skeleton data generated becomes inaccurate. In a case where the skeleton data is inaccurate, the distributed avatar video may be unnatural.

In a case where such drift errors are accumulated, for example, a recalibration or a reset process may be performed. The recalibration is a process performed on posture information obtained by processing sensor data, and the reset process is a process of adjusting parameters for generating skeleton data. In both processes, the distribution user needs to be in the initial position posture.

However, in the recalibration, the display of the avatar video temporarily disappears, and in the reset process, the avatar video that is stationary in the initial position posture is displayed. In any case, there is a disadvantage that the content becomes unnatural in a case where the avatar video is distributed live.

Furthermore, since the recalibration and the reset process described above are usually designed to be performed on the basis of determinations and operations of the distribution user or the operator side, there is room for improvement from the viewpoint of convenience.

The present inventors have conceived an embodiment of the present disclosure in view of the circumstances described above. In the information processing system according to the embodiment of the present disclosure, it is possible to easily eliminate the drift errors while avoiding the content from becoming unnatural. Hereinafter, the configuration and the operation of the distribution user terminal 20 according to such an embodiment of the present disclosure will be sequentially described in detail.

### <<2. Configuration of distribution user terminal>>

### <2-1. Overall configuration>

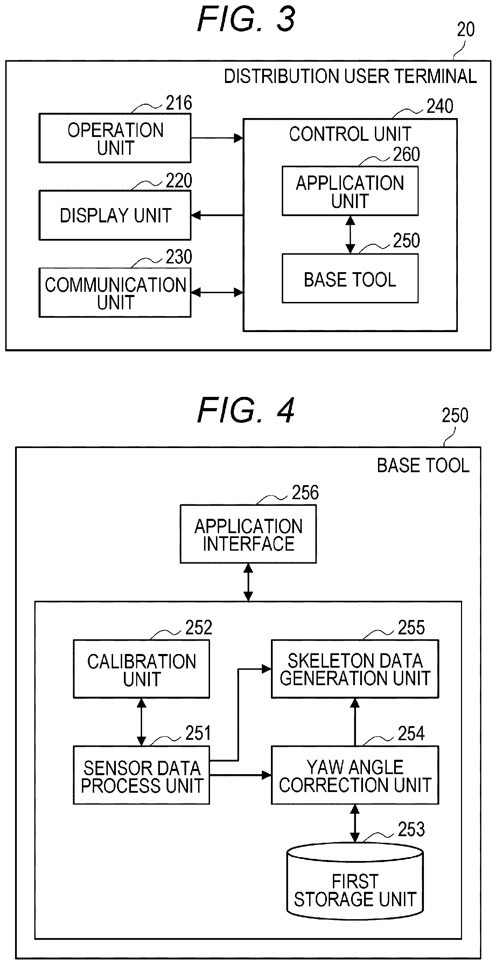

Fig. 3 is an explanatory diagram showing a configuration of the distribution user terminal 20 according to an embodiment of the present disclosure. As shown in Fig. 3, the distribution user terminal 20 according to an embodiment of the present disclosure includes an operation unit 216, a display unit 220, a communication unit 230, and a control unit 240.

The operation unit 216 is configured to be operated by the distribution user to input an instruction or information to the distribution user terminal 20. The display unit 220 displays various display screens. For example, the display unit 220 displays a display screen including the avatar video generated by the control unit 240. The communication unit 230 communicates with the distribution server 30 via the network 12. For example, the communication unit 230 transmits the avatar video generated by the control unit 240 to the distribution server 30 via the network 12.

The control unit 240 controls the overall operation of the distribution user terminal 20. In particular, the control unit 240 according to an embodiment of the present disclosure has a function of generating skeleton data of the distribution user on the basis of the sensor data received from the sensor device 10 and generating the avatar video having the posture indicated by the skeleton data. Such a function of the control unit 240 is realized by the base tool 250 and the application unit 260 shown in Fig. 3.

The base tool 250 has a function of generating skeleton data from sensor data. The base tool 250 provides the generated skeleton data to the application unit 260. Furthermore, as described in detail later, the base tool 250 according to an embodiment of the present disclosure has a function of correcting posture information (in particular, the yaw angle) of each part of the distribution user calculated from the sensor data.

The application unit 260 implements various functions in cooperation with the base tool 250. For example, the application unit 260 generates the avatar video on the basis of the skeleton data provided from the base tool 250, and requests the distribution server 30 to distribute the avatar video. Here, the application unit 260 may request the distribution server 30 to distribute a combination of the avatar video and other content data. Examples of the other content data include background data, music data, and the like. Note that the developer of the base tool 250 and the developer of the application unit 260 may be the same or different. Hereinafter, the functions of the base tool 250 and the application unit 260 will be described in more detail.

### <2-2. Functions of base tool>

Fig. 4 is an explanatory diagram showing functions of the base tool 250. As shown in Fig. 4, the base tool 250 includes a sensor data process unit 251, a calibration unit 252, a first storage unit 253, a yaw angle correction unit 254, a skeleton data generation unit 255, and an application interface 256.

### (Sensor data process unit 251)

The sensor data process unit 251 acquires sensor data indicating acceleration, angular velocity, or the like of attachment parts from the sensor device 10, and estimates position information and posture information of each attachment part on the basis of the sensor data. For example, the sensor data process unit 251 estimates position information of each attachment part by integrating acceleration data, and estimates posture information including rotation angles such as a yaw angle, a pitch angle, and a roll angle of each attachment part by integrating angular velocity data.

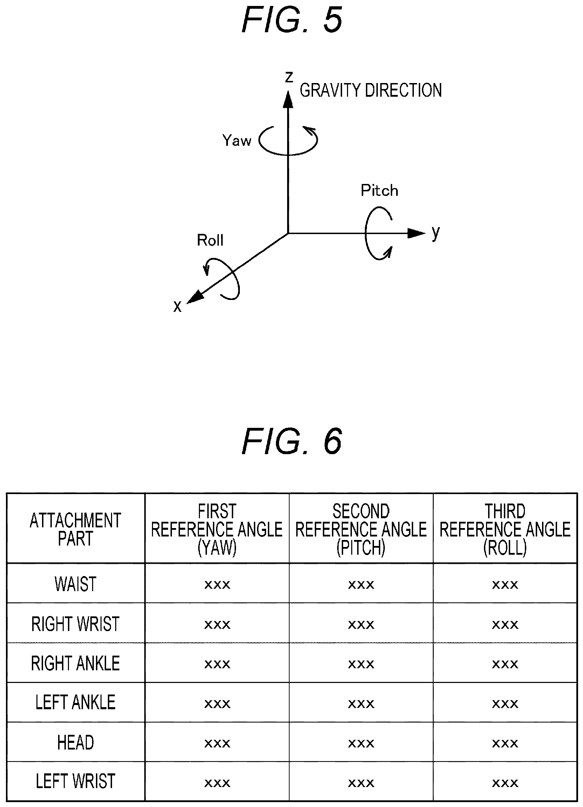

Fig. 5 is an explanatory diagram showing the yaw angle, the pitch angle, and the roll angle. As shown in Fig. 5, the yaw angle is an example of a first rotation angle that is a rotation angle around an axis in the gravity direction (z axis in Fig. 5). One of the pitch angle or the roll angle is an example of a second rotation angle that is a rotation angle around an axis orthogonal to the gravity direction (x axis in Fig. 5). The other of the pitch angle or the roll angle is an example of a third rotation angle that is a rotation angle around an axis orthogonal to both the axis in the gravity direction and the axis orthogonal to the gravity direction (y axis in Fig. 5). Note that the yaw angle of each attachment part in the present description is a relative rotation angle referenced to the yaw angle of the waist of the distribution user.

### (Calibration unit 252)

The calibration unit 252 calibrates the posture information output from the sensor data process unit 251. The calibration unit 252 may execute the calibration at the start of use of the base tool 250, or may execute the calibration according to an operation by the distribution user. At the time of calibration, the distribution user takes a posture, for example, standing firm with feet set apart and both arms down, as an initial position posture.

### (First storage unit 253)

The first storage unit 253 stores various types of information used for the operation of the base tool 250. For example, the first storage unit 253 stores posture information for registration estimated by the sensor data process unit 251 while the distribution user is taking a posture for registration.

Fig. 6 is an explanatory diagram showing a specific example of the posture information stored in the first storage unit 253. As shown in Fig. 6, the first storage unit 253 stores posture information estimated by the sensor data process unit 251 for every attachment part while the distribution user is taking a posture for registration. As the posture information, for example, the yaw angle is stored as a first reference angle, the pitch angle is stored as a second reference angle, and the roll angle is stored as a third reference angle.

These posture information is desirably posture information acquired within a predetermined time in which accumulation of drift errors is considered to be small, after calibration has been performed. Note that the posture for registration may be a unique posture that is a personalized posture for the distribution user, or may be a natural pose.

### (Yaw angle correction unit 254)

Drift errors of the pitch angle and the roll angle estimated by the sensor data process unit 251 are reduced by gravity correction. Furthermore, in a case where the difference between the pitch angle and the roll angle of a certain attachment part and the second reference angle and the third reference angle stored in the first storage unit 253 is equal to or less than the threshold, there is a possibility that the posture of the attachment part is close to the posture for registration. That is, it is considered that the original yaw angle of the attachment part is close to or equal to the first reference angle stored in the first storage unit 253.

Therefore, in a case where the conditions regarding the pitch angle and the roll angle of a certain attachment part input from the sensor data process unit 251 are satisfied, the yaw angle correction unit 254 corrects the yaw angle input from the sensor data process unit 251 for the attachment part. Specifically, in a case where the difference between the pitch angle and the roll angle of a certain attachment part and the second reference angle and the third reference angle of the attachment part is equal to or less than the threshold, the yaw angle correction unit 254 estimates that the current yaw angle of the attachment part is the first reference angle, and corrects the yaw angle of the attachment part input from the sensor data process unit 251 using the first reference angle.

The yaw angle correction unit 254 may correct the yaw angle input from the sensor data process unit 251 to the first reference angle, or correct the yaw angle input from the sensor data process unit 251 to be close to the first reference angle over time using, for example, a Kalman filter.

Note that, in the above description, an example has been described in which the yaw angle is corrected for every attachment part having the conditions satisfied, but the yaw angle correction unit 254 may correct the yaw angles for all the attachment parts in a case where all the attachment parts have the conditions satisfied.

### (Skeleton data generation unit 255)

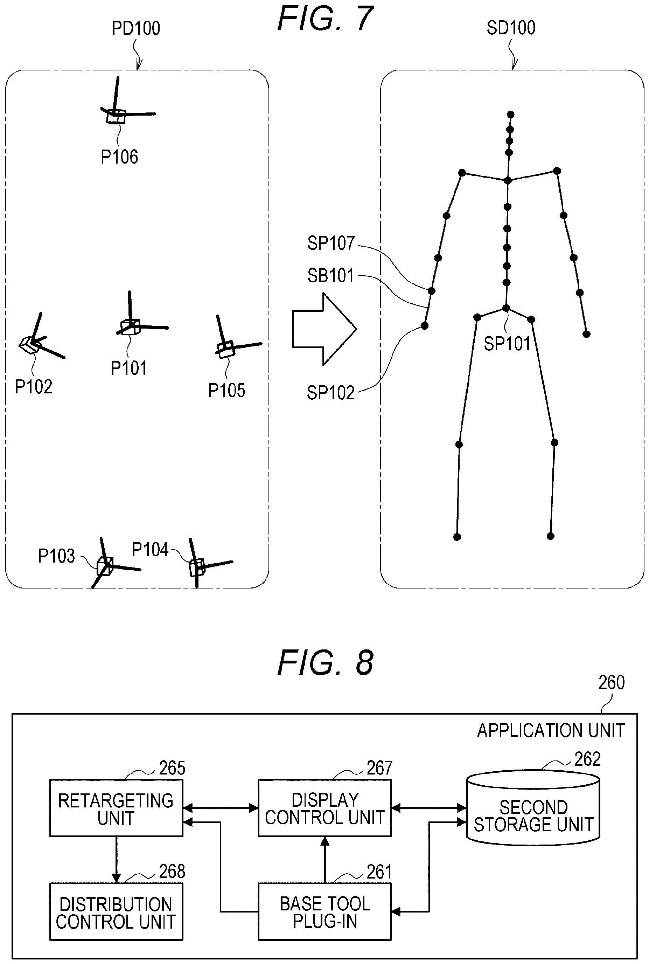

The skeleton data generation unit 255 generates skeleton data including position information and posture information of each part in the skeleton structure on the basis of the position information of each attachment part estimated by the sensor data process unit 251 and the posture information of each attachment part including the yaw angle corrected by the yaw angle correction unit 254. Hereinafter, generation of skeleton data will be described more specifically with reference to Fig. 7.

Fig. 7 is an explanatory diagram showing a specific example of generation of skeleton data. As shown in the left diagram of Fig. 7, the skeleton data generation unit 255 acquires the attachment part information PD100 including the position information and the posture information of the attachment parts P101 to P106 to which the sensor devices 10A to 10F have been attached.

Moreover, the skeleton data generation unit 255 acquires the skeleton data SD100 including the position information and the posture information of each part in the skeleton structure on the basis of the attachment part information PD100 of the attachment parts P101 to P106, as shown in the right diagram of Fig. 7. The skeleton data SD100 includes not only information of the attachment part SP101 corresponding to the attachment part P101 and the attachment part SP102 corresponding to the attachment part P102 but also information of the non-attachment part SP107.

Note that the skeleton data can include bone information (position information, posture information, and the like) in addition to the information of the part. For example, in the example shown in Fig. 7, the skeleton data SD100 may include information of the bone SB101. The skeleton data generation unit 255 can specify bone information among parts on the basis of position information and posture information of the parts in the skeleton structure.

### (Application interface 256)

The application interface 256 is an interface with the application unit 260. The application interface 256 may be configured as an application programming interface (API). For example, the application interface 256 returns the skeleton data of the distribution user to the application unit 260 in response to a request from the application unit 260. Furthermore, the application interface 256 receives an instruction to register posture information of the posture for registration from the application unit 260.

### <2-3. Functions of application unit>

The functions of the base tool 250 has been described above. Next, the functions of the application unit 260 will be described with reference to Fig. 7.

Fig. 8 is an explanatory diagram showing the functions of the application unit 260. As shown in Fig. 8, the application unit 260 includes a base tool plug-in 261, a second storage unit 262, a retargeting unit 265, a display control unit 267, and a distribution control unit 268.

### (Base tool plug-in 261)

The base tool plug-in 261 is an interface with the base tool 250. The base tool plug-in 261 receives data from the base tool 250 and converts the data into a format that can be handled by the application unit 260. For example, the base tool plug-in 261 receives, from the base tool 250, skeleton data, a notification indicating that the yaw angle described above has been corrected, and the like.

### (Second storage unit 262)

The second storage unit 262 stores various types of information used for the operation of the application unit 260. For example, the second storage unit 262 may store information regarding posture information for registration stored in the first storage unit 253 of the base tool 250.

### (Retargeting unit 265)

The retargeting unit 265 receives the skeleton data of the distribution user from the base tool plug-in 261 and retargets the skeleton data to generate the avatar video having the posture or the motion indicated by the skeleton data.

### (Display control unit 267)

The display control unit 267 generates various display screens and controls the display unit 220 to display the generated display screens. For example, the display control unit 267 generates an avatar display screen including the avatar video generated by the retargeting unit 265, and controls the display unit 220 to display the avatar display screen. Furthermore, the display control unit 267 generates a guidance screen that guides the distribution user to take the above-described posture for registration on the basis of the operation on the operation unit 216 by the distribution user or the end of the calibration, and controls the display unit 220 to display the guidance screen.

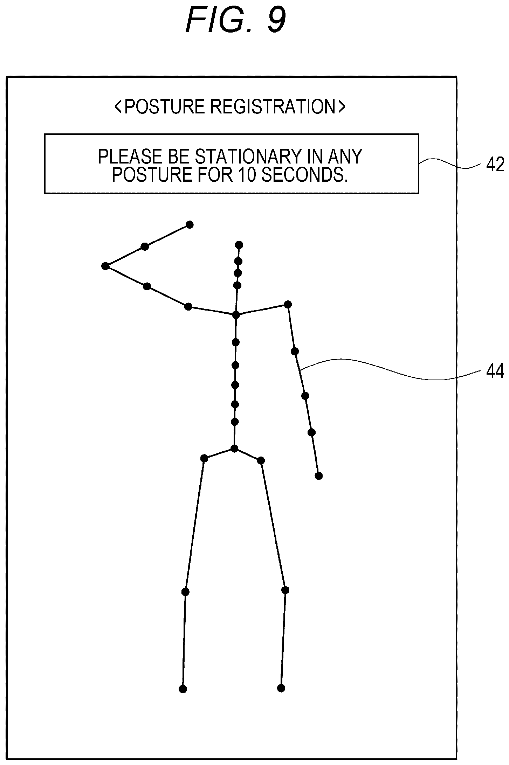

Fig. 9 is an explanatory diagram showing a specific example of the guidance screen. As shown in Fig. 9, the guidance screen includes a guidance message 42 and a skeleton data display 44. The guidance message 42 is a message requesting the distribution user to be stationary, and is "Please be stationary in any posture for 10 seconds." in the example shown in Fig. 9. The skeleton data display 44 is a display showing the skeleton data generated by the skeleton data generation unit 255 of the base tool 250 for the distribution user. Fig. 9 shows an example in which the distribution user is stationary in a posture of salute as a unique posture.

In this way, the posture information estimated by the sensor data process unit 251 of the base tool 250 while the distribution user is stationary is stored in the first storage unit 253 of the base tool 250, as described above.

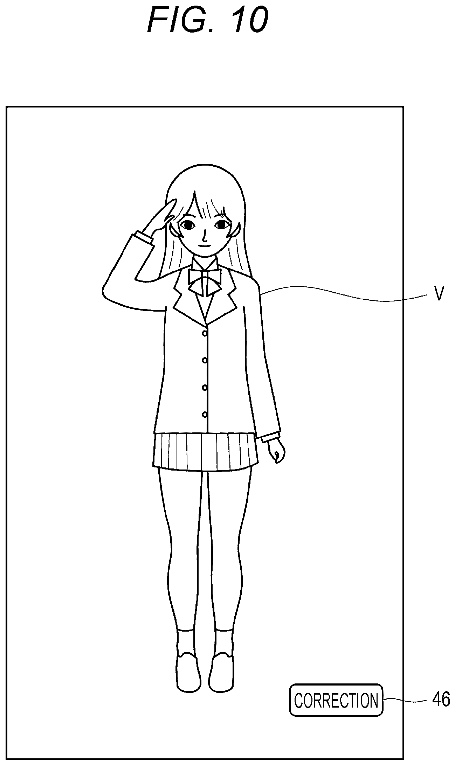

Note that, in the above description, an example has been described in which the yaw angle is automatically corrected in a case where the posture of the distribution user is close to the posture for registration, but the yaw angle may be corrected on the basis of a predetermined operation input. For example, as shown in Fig. 10, the display control unit 267 may generate an avatar display screen including the avatar video V for the distribution user, and arrange a correction button 46 on the avatar display screen. Then, when the correction button 46 is operated by the distribution user, the application unit 260 sends an execution instruction of yaw angle correction to the base tool 250, and the yaw angle correction unit 254 of the base tool 250 may correct the yaw angle on the basis of the execution instruction.

### (Distribution control unit 268)

The distribution control unit 268 transmits the avatar video generated by the retargeting unit 265 to the distribution server 30, and requests the distribution server 30 to distribute the avatar video. As a result, the avatar video is distributed to the viewing user terminal 40, and the avatar video is displayed on the viewing user terminal 40.

### <<3. Operation>>

The configuration of the information processing system according to the embodiment of the present disclosure has been described above. Next, operations of the information processing system according to the embodiment of the present disclosure will be described.

### (Registration)

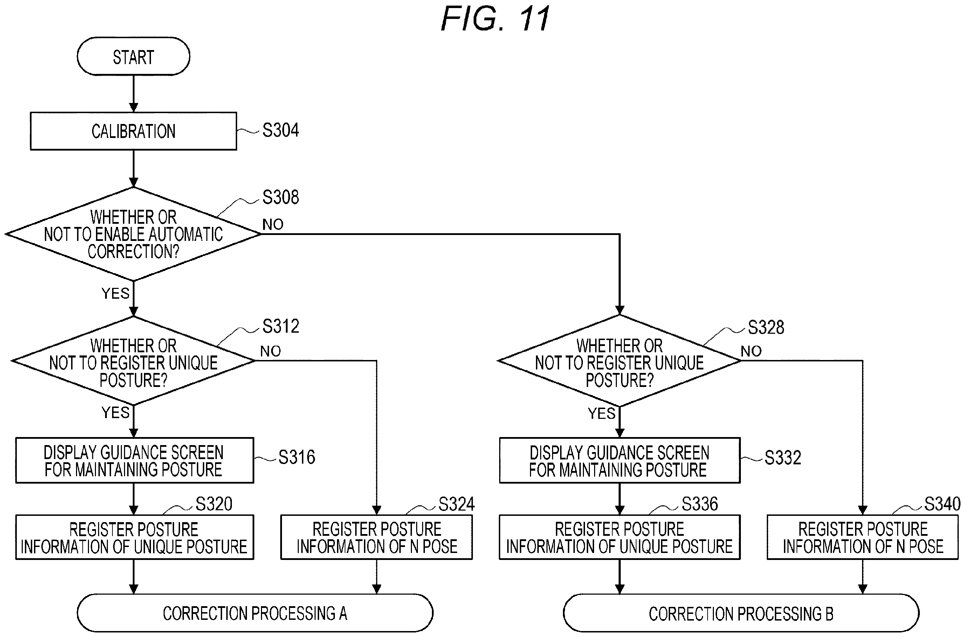

Fig. 11 is a flowchart showing a flow up to registration of posture information in the information processing system according to the embodiment of the present disclosure. First, in a state where the distribution user wears the sensor devices 10 on each part of the body and takes the initial position posture, the calibration unit 252 of the distribution user terminal 20 executes calibration for each sensor device 10 (S304).

Thereafter, the display control unit 267 controls the display unit 220 to display an automatic correction selection screen for selecting whether or not to enable the automatic correction for the yaw angle on the display unit 220, and the distribution user selects whether or not to enable the automatic correction for the yaw angle on the automatic correction confirmation screen (S308). In a case where it is selected to enable the automatic correction for the yaw angle (S308/Yes), the display control unit 267 controls the display unit 220 to display a posture registration selection screen for selecting whether or not to register a unique posture, and the distribution user selects whether or not to register the unique posture on the posture registration selection screen (S312).

In a case where it is selected to register the unique posture on the posture registration selection screen (S312/Yes), the display control unit 267 controls the display unit 220 to display the guidance screen shown in Fig. 9 for guiding the distribution user to take the unique posture as the posture for registration (S316). The distribution user maintains a stationary position in the unique posture in accordance with the guidance screen. The first storage unit 253 stores posture information of each attachment part of the distribution user estimated by the sensor data process unit 251 while the distribution user is stationary in the unique posture (S320).

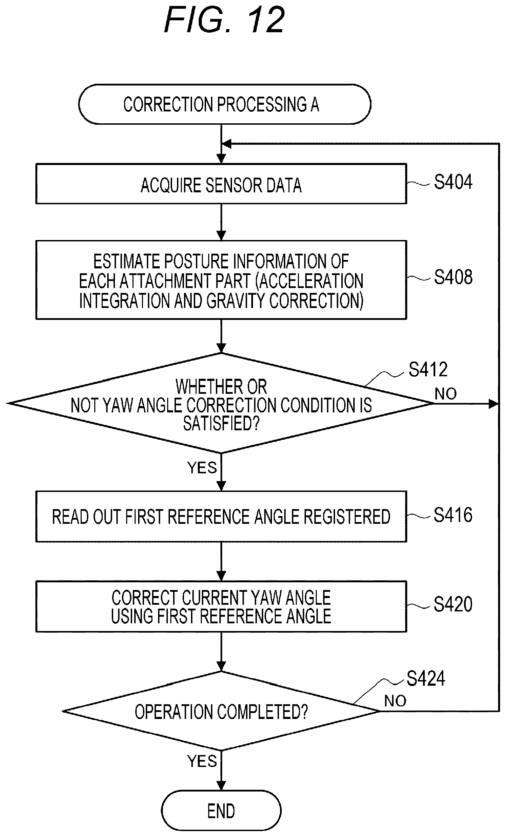

In a case where it is not selected to register the unique posture on the posture registration selection screen (S312/No), posture information of each attachment part of the distribution user estimated by the sensor data process unit 251 while the distribution user is stationary for a long time (that is, while the distribution user is taking a natural pose) is stored (S320). Note that in both S320 and S324, the sensor data process unit 251 may store a plurality of posture information. After S320 and S324, correction processing A described with reference to Fig. 12 is performed.

In a case where it is selected not to enable the automatic correction for the yaw angle on the automatic correction selection screen (S308/No), the display control unit 267 controls the display unit 220 to display a posture registration selection screen for selecting whether or not to register a unique posture, and the distribution user selects whether or not to register the unique posture on the posture registration selection screen (S328).

In a case where it is selected to register the unique posture on the posture registration selection screen (S328/Yes), the display control unit 267 controls the display unit 220 to display the guidance screen shown in Fig. 9 for guiding the distribution user to take the unique posture as the posture for registration (S332). The distribution user maintains a stationary position in the unique posture in accordance with the guidance screen. The first storage unit 253 stores posture information of each attachment part of the distribution user estimated by the sensor data process unit 251 while the distribution user is stationary in the unique posture (S336).

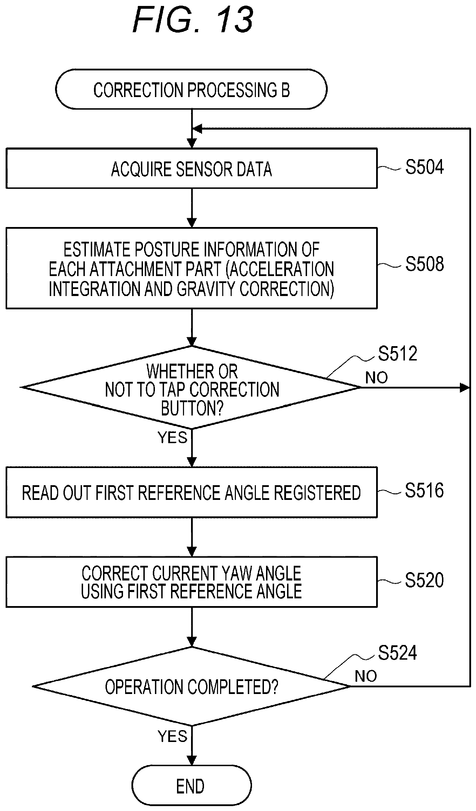

In a case where it is not selected to register the unique posture on the posture registration selection screen (S328/No), posture information of each attachment part of the distribution user estimated by the sensor data process unit 251 while the distribution user is stationary for a long time (that is, while the distribution user is taking a natural pose) is stored (S340). Note that in both S336 and S340, the sensor data process unit 251 may store a plurality of posture information. After S336 and S340, correction processing B described with reference to Fig. 13 is performed.

### (Correction processing A)

Fig. 12 is a flowchart showing correction processing A performed in a case where automatic correction is enabled. As shown in Fig. 12, first, the sensor data process unit 251 acquires sensor data from each sensor device 10 (S404). Then, the sensor data process unit 251 performs acceleration integration, gravity correction, and the like on the sensor data acquired from each sensor device 10, and estimates posture information including the yaw angle, the pitch angle, and the roll angle of each attachment part (S408).

Thereafter, the yaw angle correction unit 254 determines whether or not the yaw angle correction condition is satisfied (S412). Specifically, the yaw angle correction unit 254 determines whether or not a condition that the difference between the pitch angle and the roll angle of a certain attachment part and the second reference angle and the third reference angle of the attachment part is equal to or less than the threshold is satisfied.

In a case where the yaw angle correction condition is satisfied for the certain attachment part (S412/Yes), the yaw angle correction unit 254 reads out the first reference angle registered in the first storage unit 253 in association with the second reference angle and the third reference angle (S416). Then, the yaw angle correction unit 254 corrects the current yaw angle of the attachment part described above using the first reference angle (S420). Thereafter, the processing from S404 is repeated until the operation of the base tool 250 is completed (S424).

### (Correction processing B)

Fig. 13 is a flowchart showing correction processing B performed in a case where automatic correction is not enabled. As shown in Fig. 13, first, the sensor data process unit 251 acquires sensor data from each sensor device 10 (S504). Then, the sensor data process unit 251 performs acceleration integration, gravity correction, and the like on the sensor data acquired from each sensor device 10, and estimates posture information including the yaw angle, the pitch angle, and the roll angle of each attachment part (S508).

Thereafter, when the correction button 46 is tapped on the avatar display screen described with reference to Fig. 10 (S512/Yes), the yaw angle correction unit 254 reads out the first reference angle registered in the first storage unit 253 (S516). Here, in a case where a plurality of posture information including the first reference angle, the second reference angle, and the third reference angle is registered in the first storage unit 253, the yaw angle correction unit 254 may read out the first reference angle associated with the second reference angle and the third reference angle having the smallest difference from the current pitch angle and the current roll angle.

Then, the yaw angle correction unit 254 corrects the current yaw angle of the attachment part described above using the first reference angle (S520). Thereafter, the processing from S404 is repeated until the operation of the base tool 250 is completed (S524).

### <<4. Summary>>

According to the embodiment of the present disclosure described above, various functions and effects can be obtained. For example, in a case where the automatic correction is enabled, it is possible to automatically correct the yaw angle of each attachment part without requiring determinations and operations by the distribution user. Such an information processing system improves convenience for the distribution user.

Furthermore, according to the embodiment of the present disclosure, it is possible to register a unique posture for correction of the yaw angle. By registering a habitual posture, a determined pose, or the like taken by the distribution user as the unique posture, the yaw angle can be corrected without the distribution user having to take an unnatural posture.

Furthermore, according to the embodiment of the present disclosure, the yaw angle can be corrected for every attachment part. Therefore, when a portion of the attachment part is in the natural pose or the initial position posture, the yaw angle of the attachment part can be corrected, so that the distribution user is not necessary to take the natural pose or the initial position posture with the whole body.

Furthermore, according to the embodiment of the present disclosure, since the guidance screen for guiding the distribution user is displayed at the time of registration of the unique posture, it is possible for the distribution user to easily register the unique posture by being stationary in the unique posture in accordance with the guidance screen.

Furthermore, according to the embodiment of the present disclosure, at the time of correcting the current yaw angle, it is possible to perform correction of making the current yaw angle close to the first reference angle over time. According to such a configuration, it is possible to avoid an unnatural change in the posture of the avatar video due to a sudden change in the yaw angle.

Furthermore, according to the embodiment of the present disclosure, it is also possible to correct the yaw angle on the basis of a predetermined operation input such as tapping down the correction button 46 on the avatar display screen. According to such a configuration, it is possible to realize the correction of the yaw angle with higher accuracy by the distribution user tapping the correction button 46 when the distribution user takes the posture at the time of registration.

### <<5. Modifications>>

One embodiment of the present disclosure has been described above. Some modifications of the above-described embodiment will be described below. Note that each modification described below may be applied to the above-described embodiment alone, or may be applied to the above-described embodiment in combination. Furthermore, each modification may be applied instead of the above-described configuration, or may be additionally applied to the above-described configuration.

### <4-1. First modification>

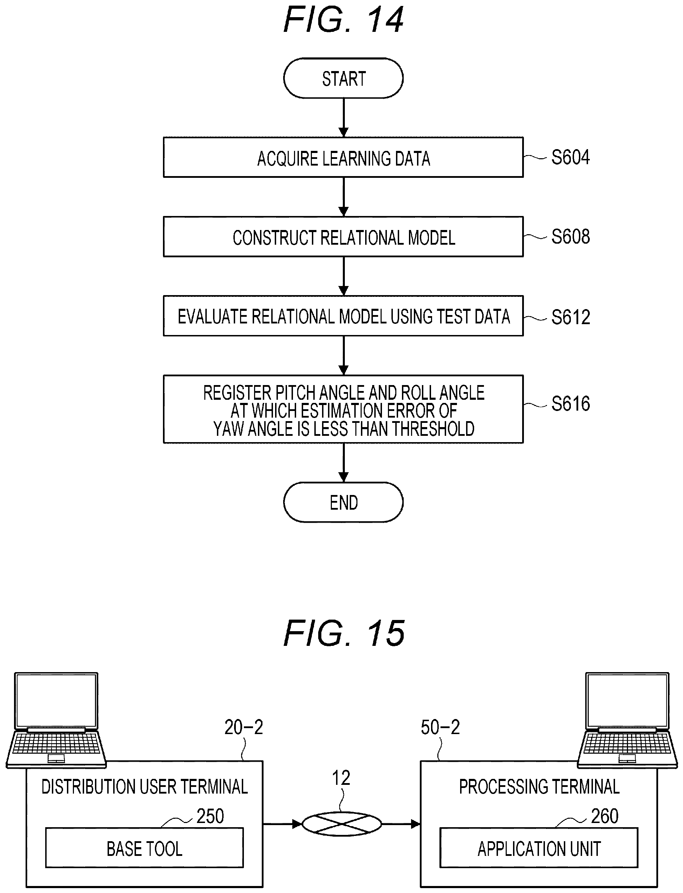

Although the example in which the base tool 250 registers the posture information of the distribution user in advance for correcting the yaw angle has been described above, the base tool 250 may register a relational model or the like between the yaw angle, and the pitch angle and the roll angle as other information for correcting the yaw angle. Hereinafter, a registration operation according to a first modification will be described with reference to Fig. 14.

Fig. 14 is a flowchart showing the registration operation according to the first modification. As shown in Fig. 14, the base tool 250 first acquires a plurality of learning data including the yaw angle, the pitch angle, and the roll angle for each attachment part (S604). The learning data may be posture information of the distribution user obtained in a period in which the influence of the drift errors of about one minute immediately after the calibration is small, or may be a general-purpose data set.

Then, the base tool 250 constructs the relational model between the pitch angle and the roll angle, and the yaw angle for each attachment part by learning based on the learning data, and registers the relational model in the first storage unit 253 (S608). Moreover, the base tool 250 evaluates the relational model using a plurality of test data including the yaw angle, and the pitch angle and the roll angle for each attachment part (S612). For example, the base tool 250 inputs the yaw angle and the pitch angle included in the test data to the relational model, and calculates a difference between the output yaw angle and the yaw angle included in the test data as an estimation error.

Subsequently, the base tool 250 specifies the yaw angle and the pitch angle at which the estimation error of the yaw angle is less than the threshold for each attachment part, and registers the yaw angle and the pitch angle in the first storage unit 253 as the second reference angle and the third reference angle (S616).

In the first modification, the yaw angle correction unit 254 estimates the yaw angle using the relational model in a case where a condition that the difference between the pitch angle and the roll angle of a certain attachment part and the second reference angle and the third reference angle of the attachment part is equal to or less than the threshold is satisfied. Then, the yaw angle correction unit 254 corrects the current yaw angle of the attachment part using the estimated yaw angle.

The first modification is useful in that the distribution user does not need to be stationary in the same posture for registration of posture information particularly in a case where a general-purpose data set is used as learning data for constructing the relational model.

### <4-2. Second modification>

In the above description, an example has been described in which the yaw angle correction unit 254 estimates the yaw angle on the basis of the current pitch angle and the current roll angle, and corrects the current yaw angle using the estimated yaw angle. However, the yaw angle correction unit 254 can also estimate the yaw angle by other methods.

For example, the base tool 250 may register the position information of each attachment part and the yaw angle of each attachment part estimated by the sensor data process unit 251 in the first storage unit 253 while the distribution user takes the posture for registration. Here, the position information may be information indicating a relative position viewed from the position of the waist of the distribution user. Furthermore, the sensor data process unit 251 may estimate the position information on the basis of the acceleration obtained by the sensor device 10, or may estimate the position information from the video of the distribution user obtained by an external camera.

Then, the yaw angle correction unit 254 may correct the current yaw angle using the yaw angle registered in the first storage unit 253 in a case where a condition that the difference between the current position information and the position information registered in the first storage unit 253 of a certain attachment part is less than a threshold is satisfied. Note that the condition may include a condition of posture information such as the pitch angle and the roll angle as in the above-described embodiment in addition to the condition of the position information, and the yaw angle correction unit 254 may correct the yaw angle in a case where both the conditions of the position information and the posture information are satisfied.

Alternatively, the sensor device 10 may include a geomagnetism sensor, and the base tool 250 may register, in the first storage unit 253, a geomagnetism value of each attachment part obtained by a geomagnetism sensor and the yaw angle of each attachment part while the distribution user takes the posture for registration. Here, the geomagnetism value may be information indicating a relative value viewed from the geomagnetism value of the waist of the distribution user.

Then, the yaw angle correction unit 254 may correct the current yaw angle using the yaw angle registered in the first storage unit 253 in a case where a condition that the difference between the current geomagnetism value and the geomagnetism value registered in the first storage unit 253 of a certain attachment part is less than a threshold is satisfied. Note that the condition may include a condition of posture information such as the pitch angle and the roll angle, a condition of position information, and the like as in the above-described embodiment, in addition to the condition of the geomagnetism value.

According to the second modification, the yaw angle can be appropriately corrected.

### <<6. Other configuration examples of information processing system>>

In the above description, the configuration example in which the distribution user terminal 20 includes the base tool 250 and the application unit 260 has been described as the first configuration example of the information processing system. However, other configuration examples are conceivable for the information processing system of the present disclosure. Hereinafter, another configuration example of the information processing system will be described.

### <5-1. Second configuration example>

Fig. 15 is an explanatory diagram showing a second configuration example of the information processing system. As shown in Fig. 15, the information processing system according to the second configuration example includes a distribution user terminal 20-2 and a processing terminal 50-2. The distribution user terminal 20-2 and the processing terminal 50-2 are connected via the network 12. The distribution user terminal 20-2 includes the base tool 250 and includes no application unit 260. The application unit 260 is implemented in the processing terminal 50-2.

In the second configuration example, the distribution user terminal 20-2 transmits skeleton data to the processing terminal 50-2. Then, the application unit 260 of the processing terminal 50-2 generates the avatar video from the skeleton data, and distributes the avatar video to the viewing user terminal 40 via the distribution server 30. In the second configuration example, the developer of the base tool 250 and the developer of the application unit 260 may be the same or different.

### <5-2. Third configuration example>

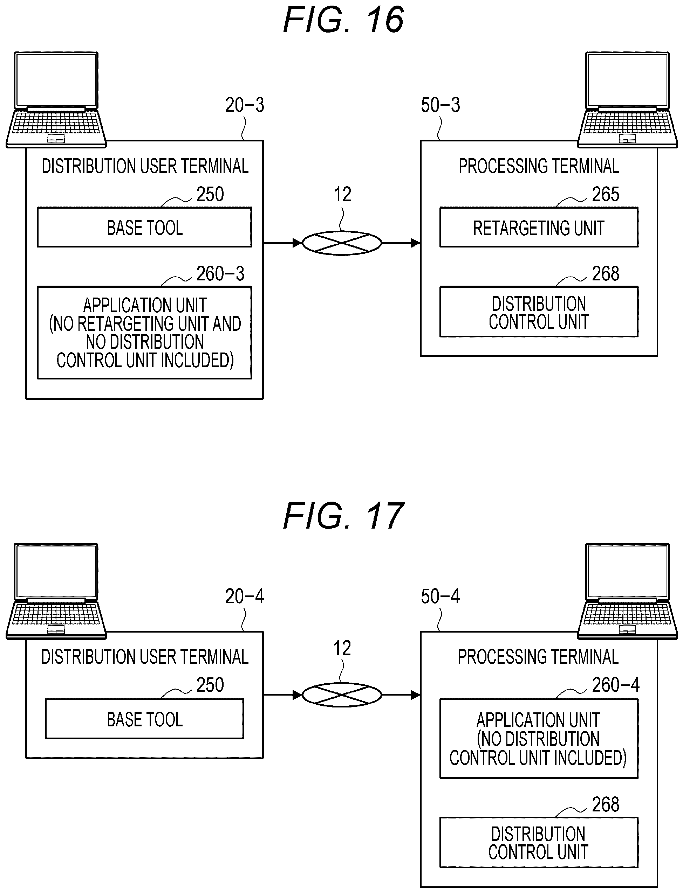

Fig. 16 is an explanatory diagram showing a third configuration example of the information processing system. As shown in Fig. 16, the information processing system according to the third configuration example includes a distribution user terminal 20-3 and a processing terminal 50-3. The distribution user terminal 20-3 and the processing terminal 50-3 are connected via the network 12. The distribution user terminal 20-3 includes the base tool 250 and an application unit 260-3. The application unit 260-3 includes no retargeting unit 265 and no distribution control unit 268 in the configuration of the application unit 260 described with reference to Fig. 8. Instead, the processing terminal 50-3 includes the retargeting unit 265 and a distribution control unit 268.

In the third configuration example, the distribution user terminal 20-3 transmits skeleton data to the processing terminal 50-3. Then, the retargeting unit 265 of the processing terminal 50-3 generates the avatar video from the skeleton data, and the distribution control unit 268 distributes the avatar video to the viewing user terminal 40 via the distribution server 30. In the third configuration example, the developer of the base tool 250, the developer of the application unit 260-3, the developer of the retargeting unit 265, and the developer of the distribution control unit 268 may be the same or different.

### <5-3. Fourth configuration example>

Fig. 17 is an explanatory diagram showing a fourth configuration example of the information processing system. As shown in Fig. 17, the information processing system according to the fourth configuration example includes a distribution user terminal 20-4 and a processing terminal 50-4. The distribution user terminal 20-4 and the processing terminal 50-4 are connected via the network 12. The distribution user terminal 20-4 includes the base tool 250. The processing terminal 50-4 includes an application unit 260-4. The application unit 260-4 includes no functions of the distribution control unit 268, and the processing terminal 50-4 separately has the functions of the distribution control unit 268.

In the fourth modification, the distribution user terminal 20-4 transmits skeleton data to the processing terminal 50-4. Then, the application unit 260-4 of the processing terminal 50-4 generates the avatar video from the skeleton data, and the distribution control unit 268 distributes the avatar video to the viewing user terminal 40 via the distribution server 30. In the fourth configuration example, the developer of the base tool 250, the developer of the application unit 260-4, and the developer of the distribution control unit 268 may be the same or different.

### <5-4. Fifth configuration example>

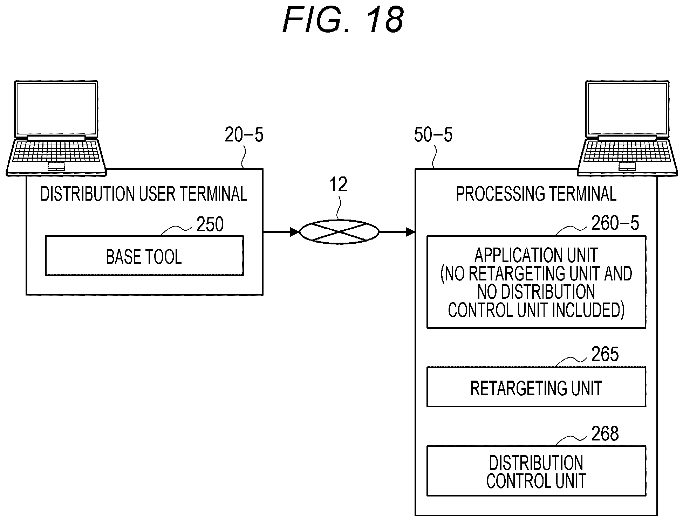

Fig. 18 is an explanatory diagram showing a fifth configuration example of the information processing system. As shown in Fig. 18, the information processing system according to the fifth configuration example includes a distribution user terminal 20-5 and a processing terminal 50-5. The distribution user terminal 20-5 and the processing terminal 50-5 are connected via the network 12. The distribution user terminal 20-5 includes the base tool 250. The processing terminal 50-5 includes an application unit 260-5. The application unit 260-5 includes no functions of the retargeting unit 265 and the distribution control unit 268, and the processing terminal 50-5 separately has the functions of the retargeting unit 265 and the distribution control unit 268.

In the fifth modification, the distribution user terminal 20-5 transmits skeleton data to the processing terminal 50-5. Then, the application unit 260-5 provides the skeleton data to the retargeting unit 265, the retargeting unit 265 generates the avatar video from the skeleton data, and the distribution control unit 268 distributes the avatar video to the viewing user terminal 40 via the distribution server 30. In the fifth configuration example, the developer of the base tool 250, the developer of the application unit 260-5, the developer of the retargeting unit 265, and the developer of the distribution control unit 268 may be the same or different.

### <5-5. Sixth configuration example>

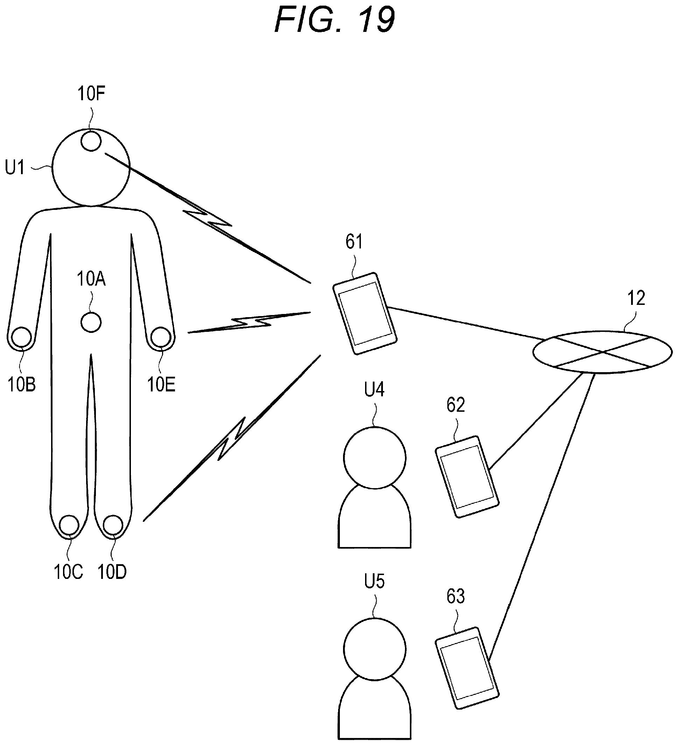

In the above description, examples have been mainly described in which the functions such as the operation unit 216, the display unit 220, the communication unit 230, and the control unit 240 are implemented on the PC-type distribution user terminal 20, but these functions may be implemented on a mobile terminal such as a smartphone. In addition, the above-described functions may be implemented in a plurality of mobile terminals in a distributed manner, or may be implemented in a distributed and repeated manner. An example in which the above-described functions are implemented in a plurality of mobile terminals in a distributed manner will be described as a sixth configuration example with reference to Fig. 19.

Fig. 19 is an explanatory diagram showing a sixth configuration example of the information processing system. As shown in Fig. 19, the information processing system according to the sixth configuration example includes a first mobile terminal 61, a second mobile terminal 62, and a third mobile terminal 63.

In the first mobile terminal 61, the functions of the control unit 240, that is, the functions of the base tool 250 and the application unit 260 are implemented. Furthermore, the first mobile terminal 61 also includes a communication unit for communicating with the second mobile terminal 62 and the third mobile terminal 63. The first mobile terminal 61 generates the avatar video of the distribution user U1 on the basis of the sensor data acquired from the sensor device 10, and transmits the avatar video to the second mobile terminal 62 and the third mobile terminal 63. Note that, Fig. 19 shows an example in which the first mobile terminal 61, the second mobile terminal 62, and the third mobile terminal 63 communicate with each other via the network 12, but the first mobile terminal 61, the second mobile terminal 62, and the third mobile terminal 63 may directly communicate with each other without going through the network 12.

The functions of the display unit 220 and the communication unit 230 are implemented on the second mobile terminal 62. The second mobile terminal 62 receives the avatar video from the first mobile terminal 61, and displays a display screen including the avatar video on the display unit 220. Therefore, the user U4 who uses the second mobile terminal 62 can confirm the avatar video.

The functions of the operation unit 216 and the communication unit 230 are implemented on the third mobile terminal 63. When the user U5 who uses the third mobile terminal 63 performs an operation for instructing the operation unit 216 to execute calibration, an operation for selecting whether or not to enable automatic correction, an operation for tapping a correction button, or the like, the third mobile terminal 63 transmits information indicating the operation to the first mobile terminal 61. The third mobile terminal 63 may also have the functions of the display unit 220 that displays a display screen including the avatar video for the operation described above.

Note that the functions of the second mobile terminal 62 and the functions of the third mobile terminal 63 may be collectively implemented in one mobile terminal. Furthermore, the second mobile terminal 62 and the third mobile terminal 63 may also have the functions of the application unit 260. In this case, the first mobile terminal 61 may transmit skeleton data instead of the avatar video to the second mobile terminal 62 and the third mobile terminal 63, and the second mobile terminal 62 and the third mobile terminal 63 may generate and display the avatar video from the skeleton data. In addition, some or all of the functions of the application unit 260 may be implemented in each mobile terminal.

As the sixth configuration example of such an information processing system, various use cases are assumed. As use cases of the sixth configuration example of the information processing system, for example, outdoor imaging, imaging while moving, imaging in a specific environment, and the like can be considered. In outdoor imaging and imaging while moving, the use of a mobile terminal eliminates the need for securing a power supply, devices for carrying equipment, and the like, making it possible to perform motion capture and data processing more lightly. Furthermore, for example, the distribution user U1 who is a performer carries the first mobile terminal 61, and the first mobile terminal 61 transmits skeleton data, an avatar video, or the like to each of the second mobile terminals 62 held by a plurality of users such as producers or directors, whereby the skeleton data, the avatar video, or the like can be immediately checked in a plurality of environments.

In addition, in imaging in a specific environment such as a live venue and a place with open space above, radio waves are likely to spread, making it difficult to receive sensor data transmitted from the sensor device 10. In this regard, when the performer wears a compact communication device such as the first mobile terminal 61, the distance between the first mobile terminal 61 and the sensor device 10 is shortened, so that the first mobile terminal 61 can receive the sensor data with high accuracy. Since a dedicated power supply is not required, the influence on the performer's clothing and performance can also be suppressed. In addition, the second mobile terminal 62, another display device, or the like can be disposed at a place away from the first mobile terminal 61.

### <<7. Hardware configuration>>

The embodiments of the present disclosure have been described above. Information processing such as yaw angle correction and skeleton data generation described above is realized by cooperation of software and hardware of the distribution user terminal 20 described below.

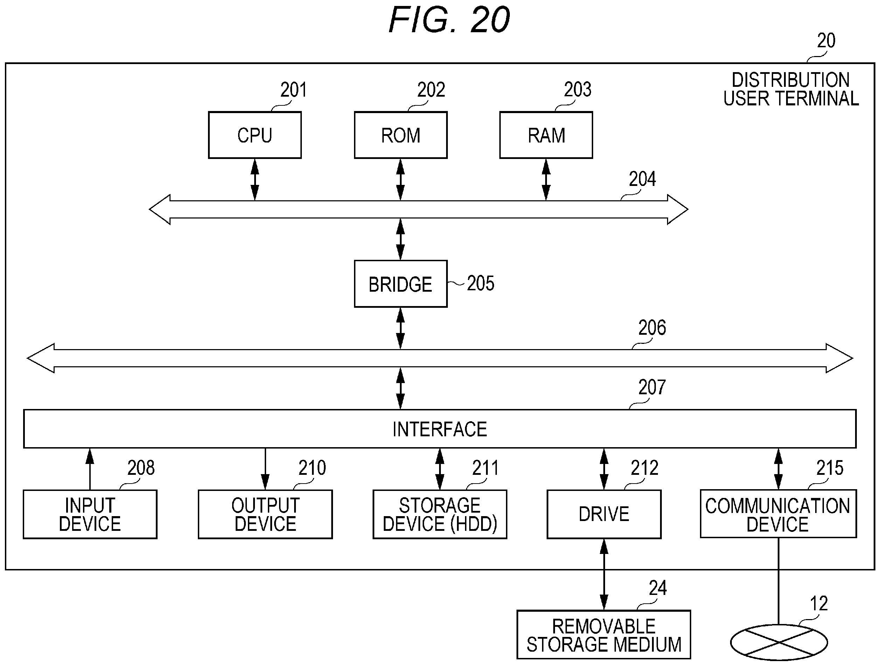

Fig. 20 is a block diagram showing a hardware configuration of the distribution user terminal 20. The distribution user terminal 20 includes a central processing unit (CPU) 201, a read only memory (ROM) 202, a random access memory (RAM) 203, and a host bus 204. In addition, the distribution user terminal 20 includes a bridge 205, an external bus 206, an interface 207, an input device 208, an output device 210, a storage device (HDD) 211, a drive 212, and a communication device 215.

The CPU 201 functions as an arithmetic processing device and a control device, and controls the overall operation in the distribution user terminal 20 according to various programs. Furthermore, the CPU 201 may also be a microprocessor. The ROM 202 stores programs, calculation parameters, and the like used by the CPU 201. The RAM 203 temporarily stores programs used in the execution of the CPU 201, parameters which change as appropriate in the execution thereof, and the like. They are connected to each other by the host bus 204 including a CPU bus and the like. Functions such as the base tool 250 and the application unit 260 described with reference to Fig. 3 can be realized by cooperation of the CPU 201, the ROM 202, the RAM 203, and software.

The host bus 204 is connected to the external bus 206 such as a peripheral component interconnect/interface (PCI) bus via the bridge 205. Note that the host bus 204, the bridge 205, and the external bus 206 are not necessarily configured separately, and their functions may be implemented on one bus.

The input device 208 includes input means for a user to input information, such as a mouse, a keyboard, a touch panel, a button, a microphone, a switch, and a lever, an input control circuit that generates an input signal on the basis of an input by the user and outputs the input signal to the CPU 201, and the like. By operating the input device 208, the user of the distribution user terminal 20 can input various data and give an instruction of processing operation to the distribution user terminal 20.

The output device 210 includes, for example, a display device such as a liquid crystal display (LCD) device, an organic light emitting diode (OLED) device, and a lamp. Moreover, the output device 210 includes an audio output device such as a speaker and headphones. The output device 210 outputs, for example, replayed content. Specifically, the display device displays various kinds of information such as replayed video data in text or images. On the other hand, the audio output device converts replayed audio data or the like into audio and outputs the audio.

The storage device 211 is a data storage device configured as an example of a storage unit of the distribution user terminal 20 according to the present embodiment. The storage device 211 may include a storage medium, a recording device that records data in the storage medium, a reading device that reads out data from the storage medium, a deletion device that deletes data recorded in the storage medium, and the like. The storage device 211 includes, for example, a hard disk drive (HDD). The storage device 211 drives a hard disk and stores programs executed by the CPU 201 and various data.

The drive 212 is a reader-writer for a storage medium, and is built in or externally attached to the distribution user terminal 20. The drive 212 reads out information recorded in a removable storage medium 24 such as a magnetic disk, an optical disk, a magneto-optical disk, or a semiconductor memory mounted thereon, and outputs the information to the RAM 203. Furthermore, the drive 212 can also write information into the removable storage medium 24.

The communication device 215 is, for example, a communication interface including a communication device or the like for connecting to the network 12. Furthermore, the communication device 215 may be a wireless local area network (LAN) compatible communication device, a long term evolution (LTE) compatible communication device, or a wire communication device that performs wired communication.

Note that the hardware configuration of the distribution user terminal 20 has been described above with reference to Fig. 20, but the hardware of the distribution server 30 and the hardware of the viewing user terminal 40 can be configured to be substantially the same as the distribution user terminal 20, and thus the description thereof will be omitted.

### <<8. Supplement>>

The preferred embodiments of the present disclosure have been described in detail above with reference to the accompanying drawings, but the present disclosure is not limited to such examples. It is apparent that a person having ordinary knowledge in the technical field to which the present disclosure belongs can devise various change examples or modification examples within the scope of the technical idea described in the claims, and it is naturally understood that they also belong to the technical scope of the present disclosure.

For example, each functional block in the base tool 250 described with reference to Fig. 4 may be implemented in a plurality of terminals in a distributed manner. Similarly, each functional block in the application unit 260 described with reference to Fig. 8 may be implemented in a plurality of terminals in a distributed manner.

Furthermore, each step in the processing of the distribution user terminal 20 of the present description is not necessarily processed in time series in the order described as in the flowchart. For example, each step in the processing of the distribution user terminal 20 may be processed in an order different from the order described as in the flowchart, or may be processed in parallel.

In addition, it is also possible to create a computer program for causing hardware such as the CPU, the ROM, and the RAM built in the distribution user terminal 20 to exert functions equivalent to the respective configurations of the distribution user terminal 20 described above. Furthermore, a storage medium storing the computer program is also provided.

Furthermore, the effects described in the present description are merely illustrative or exemplary, and are not restrictive. That is, the technology according to the present disclosure can exhibit other effects apparent to those skilled in the art from the description of the present specification, in addition to the effect described above or instead of the effect described above.

Note that the following configurations also belong to the technical scope of the present disclosure.
(1) An information processing method including:
   performing an estimation of posture information of a moving body including a first rotation angle around an axis in a gravity direction and a second rotation angle around an axis orthogonal to the gravity direction on the basis of an output from a gyro sensor attached to the moving body; and
   performing a correction of the first rotation angle in a case where a condition regarding the second rotation angle is satisfied.
(2) The information processing method according to (1), in which
   the condition includes that the second rotation angle has a predetermined relationship with a second reference angle, and
   the correction of the first rotation angle includes a correction of the first rotation angle using a first reference angle corresponding to the second reference angle.
(3) The information processing method according to (2), in which the predetermined relationship includes a relationship in which a difference between the second rotation angle and the second reference angle is equal to or less than a threshold.
(4) The information processing method according to (2) or (3), further including:
   calibrating the gyro sensor;
   estimating posture information for registration of the moving body on the basis of an output obtained from the gyro sensor while the moving body takes a posture for the registration after the calibration; and
   registering the first rotation angle included in the posture information for the registration as the first reference angle, and registering the second rotation angle included in the posture information for the registration as the second reference angle.
(5) The information processing method according to (4), further including displaying a guidance screen configured to guide the moving body to take the posture for the registration after the calibration.
(6) The information processing method according to any one of (2) to (4), in which the correction of the first rotation angle is a correction of making the one rotation angle close to the first reference angle over time.
(7) The information processing method according to any one of (1) to (6), further including performing a correction of the first rotation angle on the basis of a fact that a predetermined operation input is performed.
(8) The information processing method according to (2) or (3), further including
   constructing a relational model between the first rotation angle and the second rotation angle by learning on the basis of a plurality of learning data including the first rotation angle and the second rotation angle, in which
   the first reference angle is a first rotation angle estimated from the second rotation angle of the moving body on the basis of the relational model.
(9) The information processing method according to (8), further including:
   evaluating the relational model using a plurality of test data including the first rotation angle and the second rotation angle; and
   extracting, as the second reference angle, the second rotation angle at which an estimation error of the first rotation angle is equal to or less than a threshold in the evaluation using the plurality of test data.
(10) The information processing method according to any one of (1) to (9), in which
   the gyro sensor is attached to a plurality of parts of the moving body, and
   the correction of the first rotation angle includes the correction of the first rotation angle for a part in a case where a condition regarding the second rotation angle for the part is satisfied.
(11) The information processing method according to (1) to 10, in which
   the posture information of the moving body includes a third rotation angle around an axis orthogonal to both the axis in the gravity direction and the axis orthogonal to the gravity direction, and
   the condition is a condition regarding the second rotation angle and the third rotation angle.
(12) The information processing method according to any one of (1) to (11), further including
   estimating position information indicating a position of the moving body, in which
   the condition includes a condition regarding the position of the moving body.
(13) The information processing method according to any one of (1) to (12), in which
   a geomagnetism sensor is attached to the moving body, and
   the condition includes a condition regarding a geomagnetism value obtained from the geomagnetism sensor.
(14) An information processing apparatus, including:
   an estimation unit configured to estimate posture information of a moving body including a first rotation angle around an axis in a gravity direction and a second rotation angle around an axis orthogonal to the gravity direction on the basis of an output from a gyro sensor attached to the moving body; and
   a rotation angle correction unit configured to correct the first rotation angle in a case where a condition regarding the second rotation angle is satisfied.
(15) A program for causing a computer to function as:
   an estimation unit configured to estimate posture information of a moving body including a first rotation angle around an axis in a gravity direction and a second rotation angle around an axis orthogonal to the gravity direction on the basis of an output from a gyro sensor attached to the moving body; and
   a rotation angle correction unit configured to correct the first rotation angle in a case where a condition regarding the second rotation angle is satisfied.

### REFERENCE SIGNS LIST

- 20: Distribution user terminal
- 216: Operation unit
- 220: Display unit
- 230: Communication unit
- 250: Base tool
- 251: Sensor data process unit
- 252: Calibration unit
- 253: First storage unit
- 254: Yaw angle correction unit
- 255: Skeleton data generation unit
- 256: Application interface
- 260: Application unit
- 261: Base tool plug-in
- 262: Second storage unit
- 265: Retargeting unit
- 267: Display control unit
- 268: Distribution control unit
- 30: Distribution server
- 40: Viewing user terminal

## Claims

1. An information processing method comprising:
performing an estimation of posture information of a moving body including a first rotation angle around an axis in a gravity direction and a second rotation angle around an axis orthogonal to the gravity direction on a basis of an output from a gyro sensor attached to the moving body; and
performing a correction of the first rotation angle in a case where a condition regarding the second rotation angle is satisfied.

2. The information processing method according to claim 1, wherein
the condition includes that the second rotation angle has a predetermined relationship with a second reference angle, and
the correction of the first rotation angle includes correcting the first rotation angle using a first reference angle corresponding to the second reference angle.

3. The information processing method according to claim 2, wherein the predetermined relationship includes a relationship in which a difference between the second rotation angle and the second reference angle is equal to or less than a threshold.

4. The information processing method according to claim 2, further comprising:
calibrating the gyro sensor;
estimating posture information for registration of the moving body on a basis of an output obtained from the gyro sensor while the moving body takes a posture for the registration after the calibration; and
registering the first rotation angle included in the posture information for the registration as the first reference angle, and registering the second rotation angle included in the posture information for the registration as the second reference angle.

5. The information processing method according to claim 4, further comprising displaying a guidance screen configured to guide the moving body to take the posture for the registration after the calibration.

6. The information processing method according to claim 2, wherein the correction of the first rotation angle is a correction of making the one rotation angle close to the first reference angle over time.

7. The information processing method according to claim 1, further comprising performing a correction of the first rotation angle on a basis of a fact that a predetermined operation input is performed.

8. The information processing method according to claim 2, further comprising
constructing a relational model between the first rotation angle and the second rotation angle by learning on a basis of a plurality of learning data including the first rotation angle and the second rotation angle, wherein
the first reference angle is a first rotation angle estimated from the second rotation angle of the moving body on a basis of the relational model.

9. The information processing method according to claim 8, further comprising:
evaluating the relational model using a plurality of test data including the first rotation angle and the second rotation angle; and
extracting, as the second reference angle, the second rotation angle at which an estimation error of the first rotation angle is equal to or less than a threshold in the evaluation using the plurality of test data.

10. The information processing method according to claim 1, wherein
the gyro sensor is attached to a plurality of parts of the moving body, and
the correction of the first rotation angle includes the correction of the first rotation angle for a part in a case where a condition regarding the second rotation angle for the part is satisfied.

11. The information processing method according to claim 1, wherein
the posture information of the moving body includes a third rotation angle around an axis orthogonal to both the axis in the gravity direction and the axis orthogonal to the gravity direction, and
the condition is a condition regarding the second rotation angle and the third rotation angle.

12. The information processing method according to claim 1, further comprising
estimating position information indicating a position of the moving body, wherein
the condition includes a condition regarding the position of the moving body.

13. The information processing method according to claim 1, wherein
a geomagnetism sensor is attached to the moving body, and
the condition includes a condition regarding a geomagnetism value obtained from the geomagnetism sensor.

14. An information processing apparatus comprising:
an estimation unit configured to estimate posture information of a moving body including a first rotation angle around an axis in a gravity direction and a second rotation angle around an axis orthogonal to the gravity direction on a basis of an output from a gyro sensor attached to the moving body; and
a rotation angle correction unit configured to correct the first rotation angle in a case where a condition regarding the second rotation angle is satisfied.

15. A program for causing a computer to function as:
an estimation unit configured to estimate posture information of a moving body including a first rotation angle around an axis in a gravity direction and a second rotation angle around an axis orthogonal to the gravity direction on a basis of an output from a gyro sensor attached to the moving body; and
a rotation angle correction unit configured to correct the first rotation angle in a case where a condition regarding the second rotation angle is satisfied.
